# EUROPEAN PATENT APPLICATION

(11) **EP 2 862 521 A1**
(43) Date of publication of application: **22.04.2015**
(21) Application number: 12878826.2
(22) Date of filing: 14.06.2012
(51) Int. Cl.: A61B 17/00, A61B 17/04

(54) **PUNCTURE DEVICE**

(71) Applicant: Terumo Kabushiki Kaisha, Tokyo 151-0072 (JP)
(72) Inventor: KAWAURA, Masakatsu, Ashigarakamigun Kanagawa 259-0151 (JP); ARIURA, Shigeki, Ashigarakamigun Kanagawa 259-0151 (JP)
(74) Representative: Casalonga
(86) International application number: PCT/JP2012/065284
(87) International publication number: WO 2013/186907

(57) **Abstract**

A puncture apparatus includes: puncture means which has a puncture needle for puncturing a biological tissue located in a vicinity of a mid-urethra; and biological tissue pushing-in means which has an elongated member to be inserted into one of a urethral lumen and a vaginal cavity, the elongated member being inserted into the one of the urethral lumen and the vaginal cavity and being pressed in the insertion direction thereof, whereby the biological tissue is pushed in in the same direction as the pressing direction for the elongated member. The puncture apparatus is used by puncturing the biological tissue with the puncture needle in a state where the biological tissue has been pushed in by the biological tissue pushing-in means.

## Description

### Technical Field

The present invention relates to a puncture apparatus that is used, for example, for treatment of urinary incontinence.

### Background Art

In a patient suffering from urinary incontinence, particularly stress urinary incontinence, urine leakage occurs due to an abdominal pressure exerted during a normal exercise or by laughing, coughing, sneezing or the like. This is attributable, for example, to loosening of the pelvic floor muscle, which is a muscle for supporting the urethra, caused by childbirth or the like.

For treatment of urinary incontinence, effective is surgical therapy, in which there is used, for example, a tape-shaped implant called "sling," and the sling is placed indwelling in the body, so as to support the urethra thereby (see, for example, Patent Document 1). The present applicant has proposed a method of setting a sling to indwell in a patient's body in which a puncture needle curved for example in a circular arc shape (in the shape of letter "C") is first used to puncture, for example, a body surface, a right-hand obturator foramen, a biological tissue between the urethra and the vagina, a left-hand obturator foramen, and the body surface in this order to form a puncture hole, and the sling is passed through the puncture hole, to be placed indwelling in the body (see Patent Document 2).

Meanwhile, the biological tissue between the urethra and the vagina has a part which is suitable for puncturing by a puncture needle. This part is located between the urethra and the vagina, preferably in the vicinity of the mid-urethra. However, the position of this mid-urethra varies from person to person; for instance, in some women, the bladder neck is drooping to get comparatively near the urethral orifice, so that the mid-urethra itself is also shifted toward the urethral orifice, resulting in a shortened mid-urethra. In the case of applying the surgical treatment to such a woman, specifically, in the case of puncturing a vicinity of the mid-urethra with a puncture needle, there may be a risk, depending on the operator's skill, that the puncture needle would be deviated from the vicinity of the mid-urethra, as has been experienced in the past. Consequently, in such a situation, it would be difficult to place the sling indwelling.
Patent Document 1: Japanese Patent Laid-open No. 2010-99499
Patent Document 2: Japanese Patent Laid-open No. 2012-068400

### Disclosure of Invention

It is an object of the present invention to provide a puncture apparatus which ensures that a surgical treatment to be applied to a biological tissue located between a vaginal cavity and a urethral lumen can be carried out in an assured manner.

Such an object is achieved by the present invention as described in the following paragraphs (1) to (13).
(1) A puncture apparatus including:
   puncture means which has a puncture needle for puncturing a biological tissue located in a vicinity of a mid-urethra; and
   biological tissue pushing-in means which has an elongated member having an elongated shape to be inserted into one of a urethral lumen and a vaginal cavity, the elongated member being inserted into the one of the urethral lumen and the vaginal cavity and being pressed in an insertion direction thereof, whereby the biological tissue is pushed in in the same direction as a pressing direction for the elongated member,
   characterized in that the puncture apparatus is used by puncturing the biological tissue with the puncture needle in a state where the biological tissue has been pushed in by the biological tissue pushing-in means.
(2) The puncture apparatus as described in the aforementioned paragraph (1), wherein the biological tissue pushing-in means has a fixing part which is provided on the elongated member and which fixes the biological tissue to the elongated member from the side of the one of the urethral lumen and the vaginal cavity.
(3) The puncture apparatus as described in the aforementioned paragraph (2), wherein the fixing part is a part for sucking the biological tissue to bring the biological tissue into secure contact with the elongated member.
(4) The puncture apparatus as described in the aforementioned paragraph (3), wherein the elongated member is a hollow body and is connected to suction means for applying suction to the inside of the hollow body, and
   the elongated member is formed with at least one suction port opening in an outer circumferential surface thereof, the suction port functioning as the fixing part and communicating with the inside of the hollow body, with the biological tissue being drawn toward and brought into secure contact with the elongated member by a suction force generated at the suction port when the suction means is operated.
(5) The puncture apparatus as described in the aforementioned paragraph (4), wherein the one of the urethral lumen and the vaginal cavity is the urethral lumen, and
   the biological tissue pushing-in means has a seal member for airtight sealing of the urethral lumen in a state where the elongated member is inserted in the urethral lumen.
(6) The puncture apparatus as described in any one of the aforementioned paragraphs (1) to (5), wherein the biological tissue pushing-in means has an auxiliary elongated member having an elongated shape to be inserted into the other of the urethral lumen and the vaginal cavity, the auxiliary elongated member being inserted into the other of the urethral lumen and the vaginal cavity and being pressed in an insertion direction thereof, whereby the biological tissue is pushed in together with the elongated member.
(7) The puncture apparatus as described in the aforementioned paragraph (6), wherein the biological tissue pushing-in means has an auxiliary fixing part which is provided on the auxiliary elongated member and which fixes the biological tissue to the auxiliary elongated member from the side of the other of the urethral lumen and the vaginal cavity.
(8) The puncture apparatus as described in the aforementioned paragraph (7), wherein the auxiliary fixing part is a part for sucking the biological tissue to bring the biological tissue into secure contact with the auxiliary elongated member.
(9) The puncture apparatus as described in the aforementioned paragraph (8), wherein the auxiliary elongated member is a hollow body and is connected to auxiliary suction means for applying suction to the inside of the hollow body, and
   the auxiliary elongated member is formed with at least one auxiliary suction port opening in an outer circumferential surface thereof, the auxiliary suction port functioning as the auxiliary fixing part and communicating with the inside of the hollow body constituting the auxiliary elongated member, with the biological tissue being drawn toward and brought into secure contact with the auxiliary elongated member by a suction force generated at the auxiliary suction port when the auxiliary suction means is operated.
(10) The puncture apparatus as described in any one of the aforementioned paragraphs (6) to (9), further including interlock means for interlocking the elongated member and the auxiliary elongated member so as to permit the elongated member and the auxiliary elongated member to come closer to and away from each other.
(11) The puncture apparatus as described in the aforementioned paragraph (10), wherein the biological tissue pushing-in means includes a fixing part which is provided on the elongated member and which fixes the biological tissue to the elongated member from the side of the one of the urethral lumen and the vaginal cavity, and an auxiliary fixing part which is provided on the auxiliary elongated member and which fixes the biological tissue to the auxiliary elongated member from the side of the other of the urethral lumen and the vaginal cavity; and
   the interlock means is so configured as to be capable of spacing the elongated member and the auxiliary elongated member away from each other and maintaining the spaced-apart state, in a state where the biological tissue is fixed to the elongated member by the fixing part and where the biological tissue is fixed to the auxiliary elongated member by the auxiliary fixing part.
(12) The puncture apparatus as described in the aforementioned paragraph (11), further including a maker which, when the elongated member and the auxiliary elongated member are spaced away from each other, indicates the separated distance between the elongated member and the auxiliary elongated member.
(13) The puncture apparatus as described in any one of the aforementioned paragraphs (1) to (12), wherein the puncture needle is curved in a circular arc shape, and the puncture means has a rotational support member for supporting the puncture needle so as to permit rotary movement of the puncture needle about a center of the circular arc.

### Brief Description of Drawings

[FIG. 1]

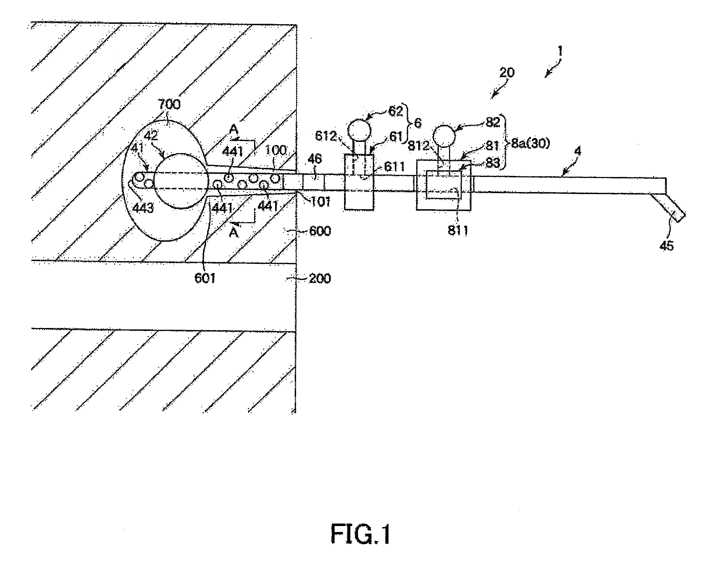

   FIG. 1 is a vertically sectional side view for sequentially illustrating a method of using a puncture apparatus of the present invention (a first embodiment).
[FIG. 2]

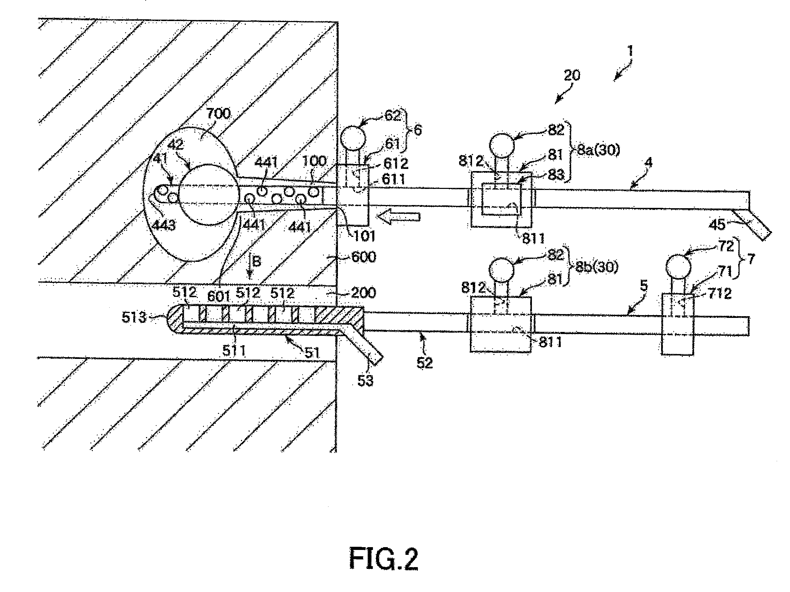

   FIG. 2 is a vertically sectional side view for sequentially illustrating the method of using the puncture apparatus of the present invention (the first embodiment).
[FIG. 3]

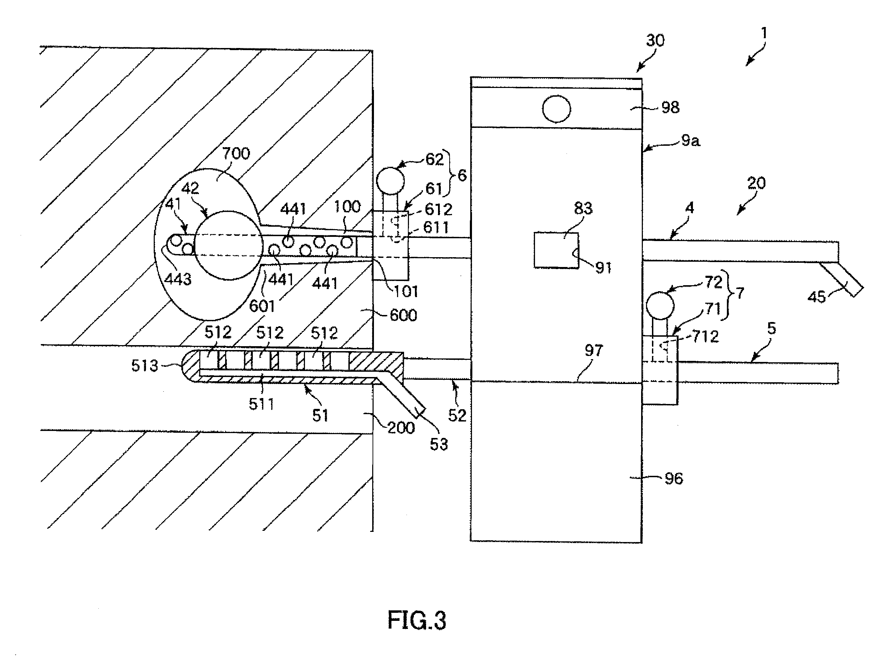

   FIG. 3 is a vertically sectional side view for sequentially illustrating the method of using the puncture apparatus of the present invention (the first embodiment).
[FIG. 4]

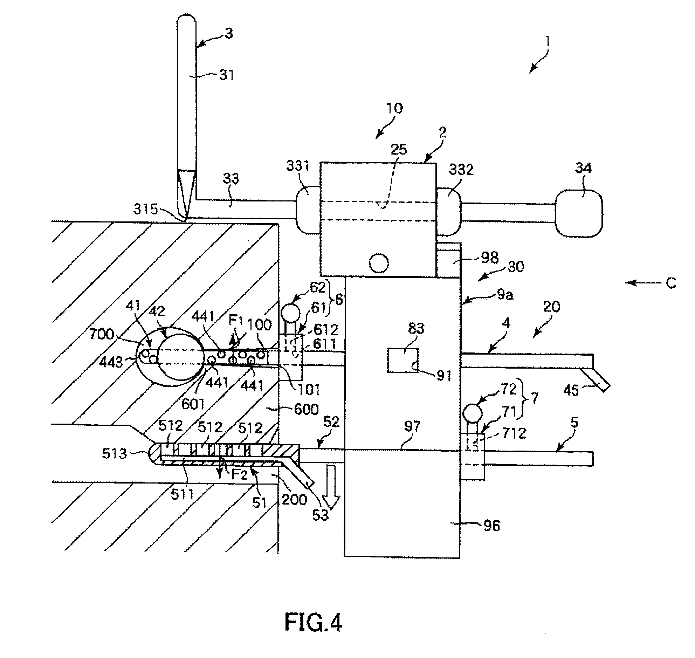

   FIG. 4 is a vertically sectional side view for sequentially illustrating the method of using the puncture apparatus of the present invention (the first embodiment).
[FIG. 5]

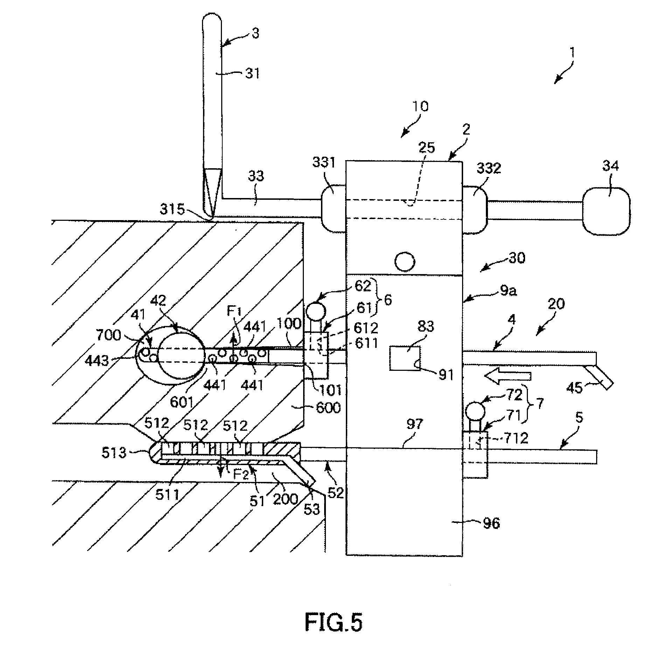

   FIG. 5 is a vertically sectional side view for sequentially illustrating the method of using the puncture apparatus of the present invention (the first embodiment).
[FIG. 6]

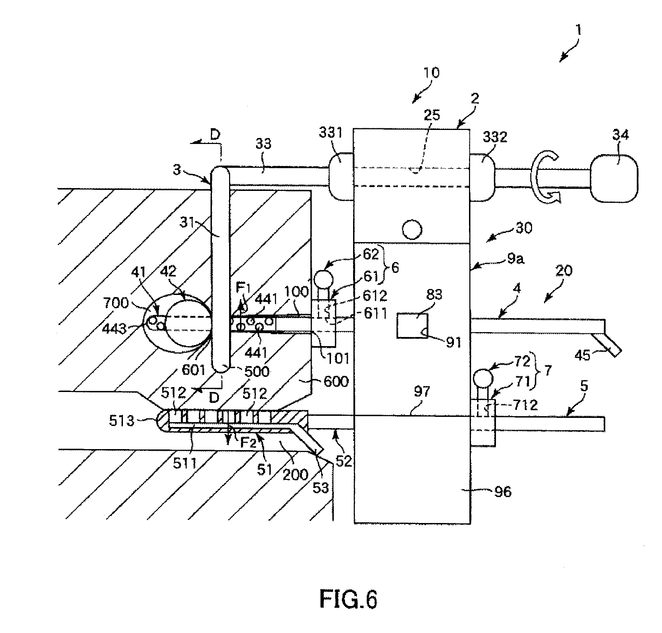

   FIG. 6 is a vertically sectional side view for sequentially illustrating the method of using the puncture apparatus of the present invention (the first embodiment).
[FIG. 7]

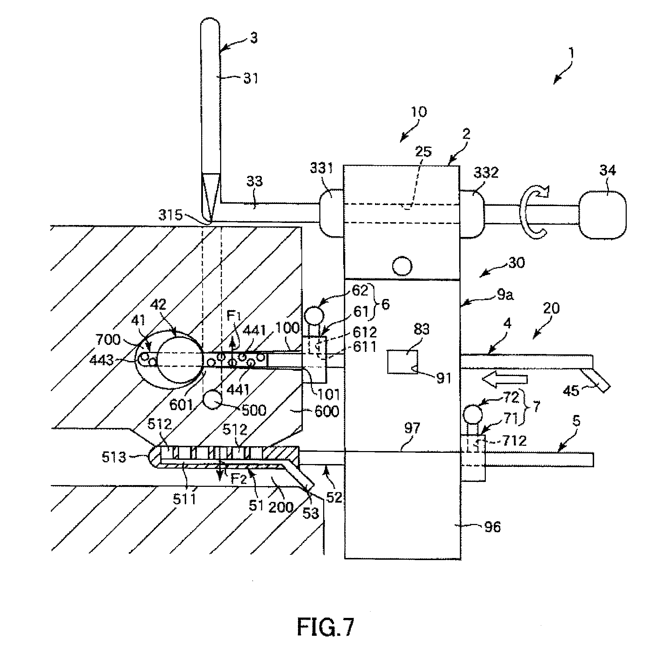

   FIG. 7 is a vertically sectional side view for sequentially illustrating the method of using the puncture apparatus of the present invention (the first embodiment).
[FIG. 8]

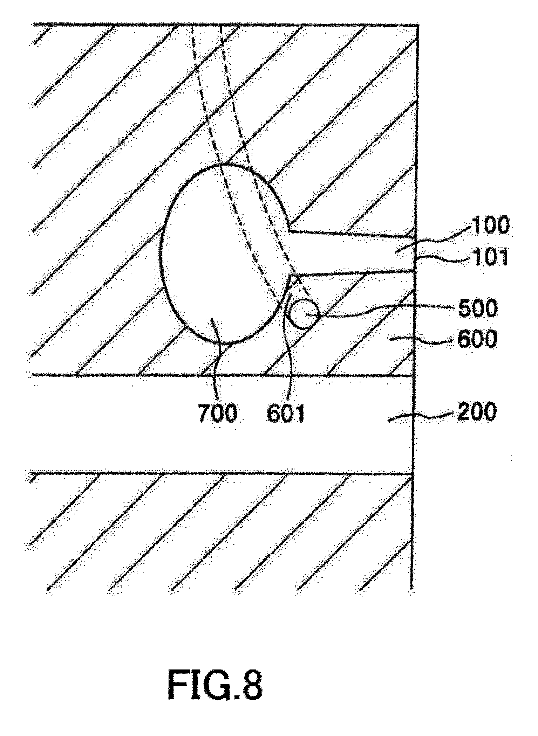

   FIG. 8 is a vertically sectional side view for sequentially illustrating the method of using the puncture apparatus of the present invention (the first embodiment).
[FIG. 9]

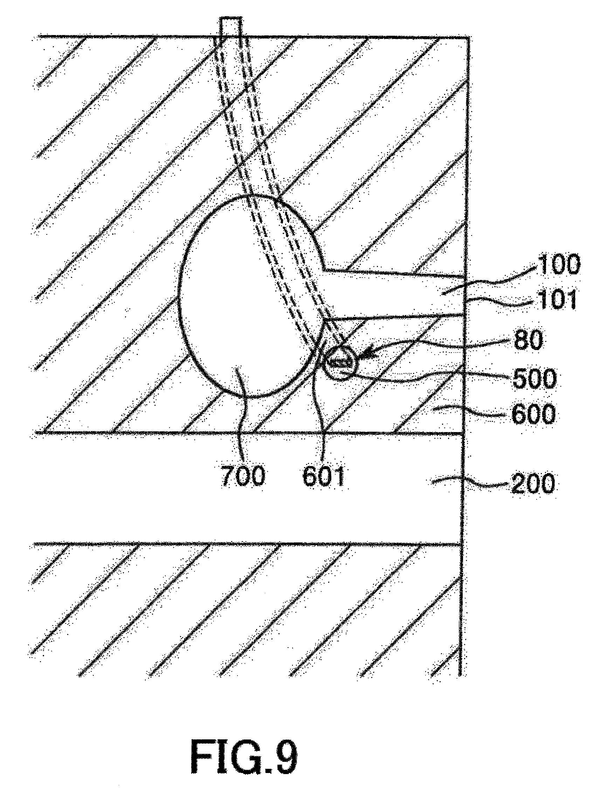

   FIG. 9 is a vertically sectional side view for sequentially illustrating the method of using the puncture apparatus of the present invention (the first embodiment).
[FIG. 10]

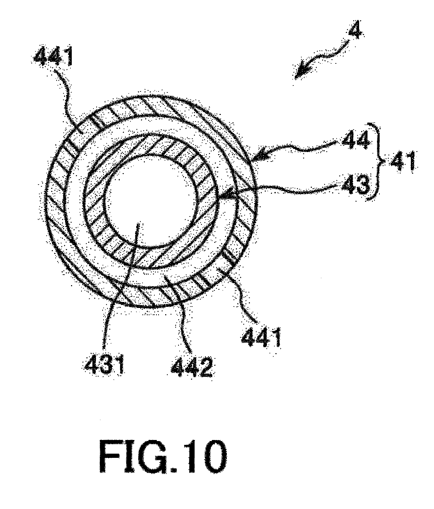

   FIG. 10 is a sectional view taken along line A-A in FIG. 1.
[FIG. 11]

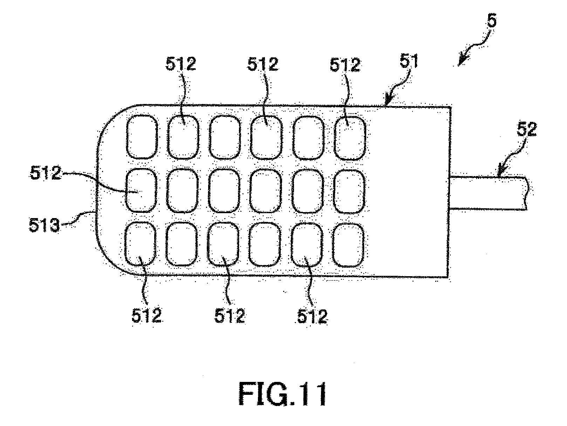

   FIG. 11 is a view taken in the direction of arrow B in FIG. 2.
[FIG. 12]

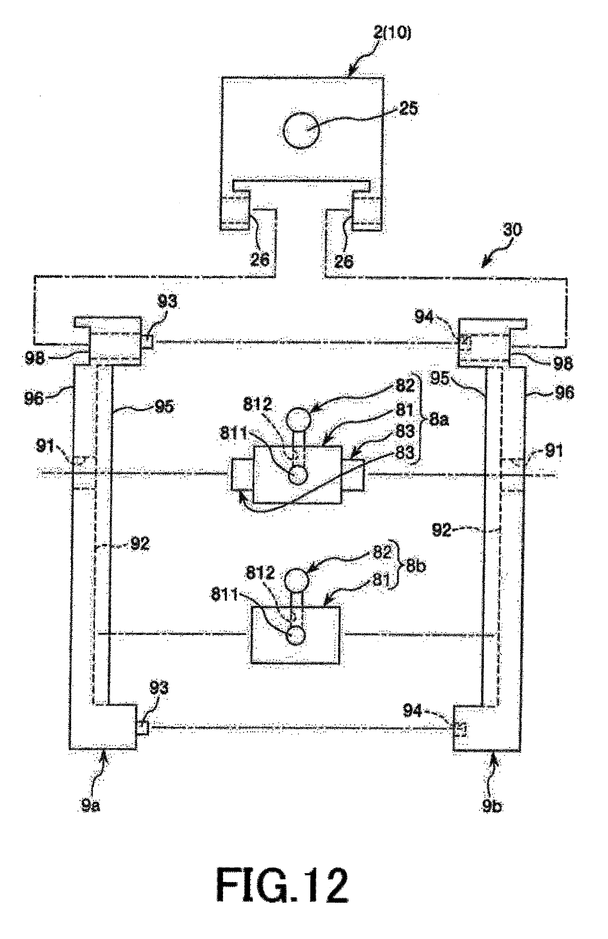

   FIG. 12 is a view taken in the direction of arrow C in FIG. 4.
[FIG. 13]

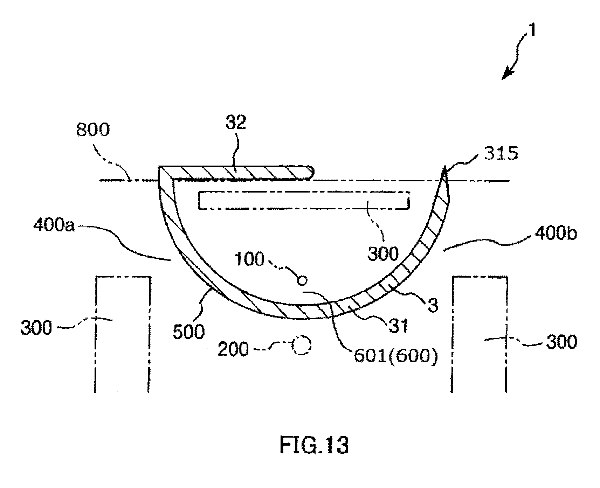

   FIG. 13 is a sectional view taken along line D-D in FIG. 6.
[FIG. 14]

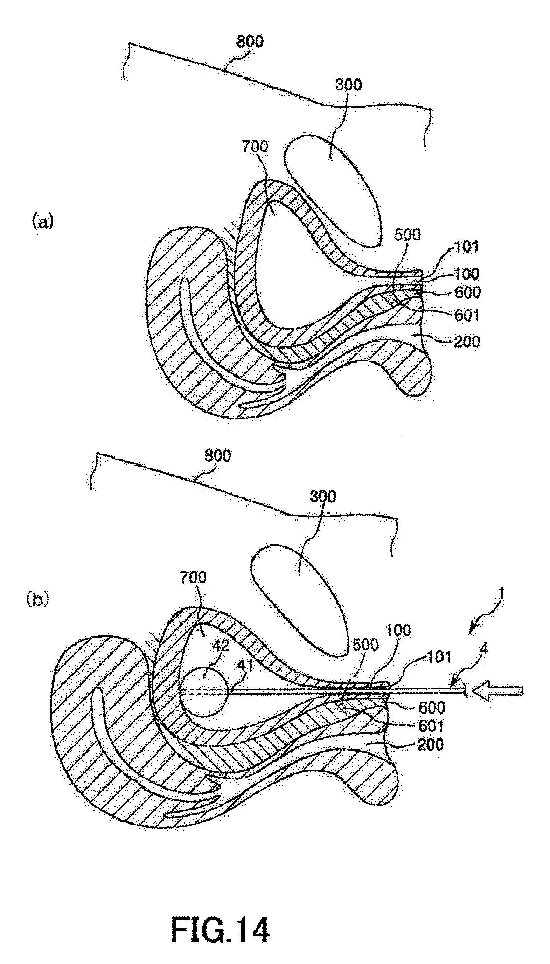

   FIG. 14 shows vertically sectional side views for sequentially illustrating a method of using a puncture apparatus of the present invention (a second embodiment).
[FIG. 15]

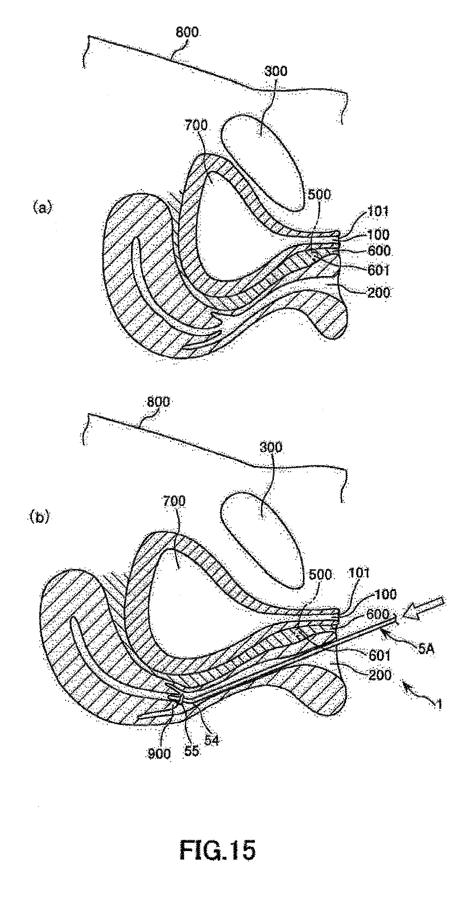

   FIG. 15 shows vertically sectional side views for sequentially illustrating a method of using a puncture apparatus of the present invention (a third embodiment).

### Best Modes for Carrying Out the Invention

Hereafter, the puncture apparatus of the present invention will be described in detail based on preferred embodiments shown in the attached drawings.

### <First Embodiment>

FIGS. 1 to 9 are vertically sectional side views for sequentially illustrating a method of using a puncture apparatus of the present invention (a first embodiment); FIG. 10 is a sectional view taken along line A-A in FIG. 1; FIG. 11 is a view taken in the direction of arrow B in FIG. 2; FIG. 12 is a view taken in the direction of arrow C in FIG. 4; and FIG. 13 is a sectional view taken along line D-D in FIG. 6. Note that hereinafter the right side in FIGS. 1 to 9 and 11 (and in FIGS. 14 and 15, as well) will be referred to as "proximal (end)," and the left side as "distal (end)," for convenience of explanation. In addition, the upper side in FIGS. 1 to 8, 12 and 13 (and in FIGS. 14 and 15, as well) will be referred to as "upper (side)" or "above," and the lower side as "lower (side)" or "downward."

The puncture apparatus 1 shown in these drawings is a medical instrument to be used for treatment of female urinary incontinence, that is, to be used for burying an implant (in vivo indwelling instrument) in a biological tissue 600 located between a urethral lumen (one of the urethral lumen and a vaginal cavity) 100 and a vaginal cavity (the other of the urethral lumen and the vaginal cavity) 200.

The urethral lumen 100 and the vaginal cavity 200 are located adjacent to each other, with the biological tissue 600 interposed therebetween (see FIGS. 1 to 9). The biological tissue 600 includes a wall portion (urethral wall) defining the urethral lumen 100, a wall portion (vaginal wall) defining the vaginal cavity 200, a connective tissue existing between the wall portions, and the like.

The implant is an instrument which is buried in the biological tissue 600 so as to support the urethra in the manner of pulling the urethra in a direction for spacing away from the vaginal wall, for the treatment of female urinary incontinence (see FIG. 9). This makes it possible to support the urethra and, hence, to prevent urine leakage.

As the implant, there can be used, for example, an elongated member which is flexible. In this embodiment, the implant is composed of a band 80. This band 80 is called "sling."

In addition, the size of the band 80 is not particularly limited, but is set as required. The width is preferably about 3 to 15 mm, and the thickness is preferably about 0.2 to 2 mm.

Besides, the material constituting the band 80 is not specifically restricted. There can be used, for example, various resin materials that are biocompatible.

Note that while the implant is composed of a single band 80 in this embodiment, this is not restrictive, and the implant may be composed of a plurality of bands 80, for example.

In addition, the implant is not limited to the band or bands 80; other flexible elongated bodies such as, for example, strings, cords and the like can be used as the implant. In the case where a string or cord is used as the implant and where the string or cord is circular in cross-sectional shape, its diameter is preferably about 0.2 to 5 mm.

The puncture apparatus 1 is capable of forming a through-hole (puncture hole) 500 in the biological tissue 600, for burying the band 80 in the biological tissue 600. Then, by inserting and passing the band 80 into and through the through-hole 500, the band 80 can be buried.

The puncture apparatus 1 includes puncture means 10 for puncturing the biological tissue 600, biological tissue pushing-in means 20 for pushing in the biological tissue 600, and interlock means 30.

As shown in FIGS. 4 to 7, the puncture means 10 is composed of a puncture member 3, and a rotational support member 2 for supporting the puncture member 3 in a rotatable manner.

The puncture member 3 has a puncture needle 31 for puncturing the biological tissue 600, a shaft portion 33, and an interlock portion 32 that interlocks the puncture needle 31 and the shaft portion 33 (see FIGS. 4 to 7 and 13).

The puncture needle 31 is so designed as to puncture the biological tissue 600 located in a vicinity 601 of a mid-urethra. In this embodiment, the puncture needle 31 is curved in the shape of letter "C," or in a circular arc shape, and has a sharp needle tip 315 at one end thereof. With the needle tip 315, the biological tissue 600 can be punctured in an assured manner. Note that while the needle tip 315 of the puncture needle 31 is oriented counterclockwise in the configuration shown in FIG. 13, this is not restrictive, and the needle tip 31 may be oriented clockwise.

In addition, the center angle of the circular arc of the puncture needle 31 is not particularly limited, but is set as required according to various conditions. In practice, the center angle is so set that, at the time of puncturing the biological tissue 600 by the puncture needle 31, the puncture needle 31 is able to enter the patient's body via a body surface 800 on one side, to pass the biological tissue 600, and to protrude to the outside of the patient's body via the body surface 800 on the other side. Such a center angle is, for example, preferably 120 to 270 degrees, more preferably 160 to 230 degrees, and further preferably 180 to 210 degrees.

The shaft portion 33 is rectilinear in shape, and is located on a center line of the circular arc shape of the puncture needle 31.

The interlock portion 32 is a portion which is rectilinear in shape and which interlocks the other end of the puncture needle 31 and the tip of the shaft portion 33.

Note that the material constituting the puncture member 3 is not specifically restricted, and, for example, various metallic materials such as stainless steels, aluminum or aluminum alloys, titanium or titanium alloys, etc. can be used as the material. In addition, the puncture needle 31 may be either a solid body or a hollow body.

The rotational support member 2 is a member for supporting the puncture needle 31 so as to permit rotary movement of the puncture needle 31 about the shaft portion 33.

As shown in FIGS. 4 to 12, the rotational support member 2 is block-like in shape, and is formed with a pass-through hole 25 piercing therethrough. With the shaft portion 33 passed through the pass-through hole 25, the puncture needle 31 can be put into the rotary movement.

Note that the shaft portion 33 is formed on the distal side and the proximal side thereof with a flange 331 and a flange 332, with the rotational support member 2 interposed therebetween. The flanges 331 and 332 inhibit the shaft portion 33 from moving in the axial direction relative to the rotational support member 2. Accordingly, the shaft portion 33 can be prevented from being pulled out of the pass-through hole 25.

In addition, the material constituting the rotational support member 2 is not specifically restricted; for example, various resin materials can be used as the material.

The biological tissue pushing-in means 20 includes a urethral-insertion member (elongated member) 4 which is elongated in shape and serves as a first elongated member to be inserted into the urethral lumen 100, a vaginal-insertion member (auxiliary elongated member) 5 which is elongated in shape and serves as a second elongated member to be inserted into the vaginal cavity 200, and a seal member 6 for airtight sealing of the urethral lumen 100.

As shown in FIGS. 1 to 7, the urethral-insertion member 4 is a balloon catheter that has a main body portion 41 and a balloon 42 provided at a distal portion of the main body portion 41.

As shown in FIG. 10, the main body portion 41 is composed of a structural body (hollow body) with a double-tube structure including an inner tube 43 and an outer tube 44. Note that various metallic materials such as aluminum, aluminum alloys, stainless steels, etc. or various resin materials can be used as a material or materials constituting the inner tube 43 and the outer tube 44.

The balloon 42, which can be expanded and contracted, is fixed to a distal outer circumferential portion of the outer tube 44. The inside of the balloon 42 communicates with a lumen portion 431 of the inner tube 43. A working fluid can be supplied through the lumen portion 431 into the balloon 42. By this, the balloon 42 can be expanded. Besides, with the working fluid discharged from the expanded balloon 42 by way of the lumen portion 431, the balloon 42 can be contracted. As described above, the lumen portion 431 functions as a lumen through which the working fluid passes. Note that the working fluid is not specifically restricted, and, for example, air, water and the like can be used as the working fluid.

Such a balloon 42 is composed of a membrane formed in a tubular shape, and is installed in position with its proximal portion and its distal portion firmly attached to a distal outer circumferential portion of the outer tube 44 in an airtight manner. When the puncture apparatus 1 is used, the balloon 42 is inserted into a patient's bladder 700, and, in its expanded state, is caught on the vicinity of the bladder neck constituting part of the biological tissue 600. By this, the position of the urethral-insertion member 4 in the longitudinal direction relative to the biological tissue 600 can be restricted. Consequently, the urethral-insertion member 4 can be securely prevented from being unwillingly pulled out of the urethral lumen 100.

Note that the method for firmly attaching the balloon 42 to the outer tube 44 is not specifically restricted, and examples of the usable method include methods by welding (thermal welding, high-frequency welding, ultrasonic welding, etc.) and methods by adhesion (adhesion with an adhesive or solvent).

In addition, the material constituting the balloon 42 is not particularly limited, and examples of the applicable material include polyester resins such as polyethylene terephthalate, polyethylene naphthalate, polybutylene terephthalate, polybutylene naphthalate, etc., polyester elastomers containing these polyester resins, olefin resins such as polyethylene, polypropylene, etc., crosslinked products (particularly, products of crosslinking by irradiation with electron rays) of the olefin resins, polyamide resins such as nylon 11, nylon 12, nylon 610, etc., polyamide elastomers containing these polyamide resins, polyurethane resins, ethylenevinyl acetate copolymer and crosslinked products these polymers, as well as polymer blends, polymer alloys and the like containing at least one of these polymers.

In addition, the outer tube 44 is provided in a distal outer circumferential portion thereof with a plurality of suction ports 441, located so as to avoid the part where the balloon 42 is disposed. These suction ports 441 are arranged in the longitudinal direction and the circumferential direction of the outer tube 44, and function as a fixing part for fixing the biological tissue 600 onto the urethral-insertion member 4 from the side of the urethral lumen 100.

As shown in FIG. 10, each of the suction ports 441 is composed of a through-hole which is formed to penetrate the tube wall of the outer tube 44 and which communicates with a lumen portion 442 of the outer tube 44. Suction means (not shown) such as a wall suction provided in an operating room, for example, is connected to a port 45 disposed at a proximal portion of the outer tube 44, whereby suction can be applied to the lumen portion 442 of the outer tube 44. By this suction, namely, by operating the suction means, there is generated a suction force F₁ with which the biological tissue 600 is drawn toward and brought into secure contact with the urethral-insertion member 4 at each suction port 441, whereby the biological tissue 600 is fixed assuredly (see FIGS. 4 to 7).

Note that while the shape of the suction port 441 in side view is a circular shape in this embodiment, this is not restrictive, and the shape may be an elliptic shape or a rectangular shape, for example.

In addition, a distal end surface 443 of the outer tube 44 is rounded. This makes it possible to securely prevent the inside of the urethral lumen 100 from being damaged by the distal end surface 443 of the outer tube 44 during the time when the urethral-insertion member 4 is being inserted into the urethral lumen 100.

As shown in FIGS. 2 to 7 and 11, the vaginal-insertion member 5 includes a flat-shaped head part 51, and a neck part 52 connected to a proximal portion of the head part 51. Note that various metallic materials such as aluminum, aluminum alloys, stainless steels, etc. or various resin materials can be used as material or materials constituting the head part 51 and the neck part 52.

As shown in FIGS. 2 to 7, the head part 51 is a hollow body, having a lumen portion 511. Besides, the head part 51 is provided with a plurality of suction ports 512 in its surface (outer circumferential portion) that faces the biological tissue 600 when the head part 51 is inserted in the vaginal cavity 200. These suction ports 512 are arranged in the longitudinal direction and the width direction of the head part 51 (see FIG. 11), and constitute a part functioning as a fixing part (auxiliary fixing part) for fixing the biological tissue 600 onto the head part 51 from the side of the vaginal cavity 200.

Each of the suction ports 512 is composed of a through-hole which is formed to penetrate a wall portion of the head part 51 and which communicates with the lumen portion 511 of the head part 51. The aforementioned wall suction (auxiliary suction means) connected also to the port 45 of the urethral-insertion member 4 is connected to a port 53 disposed at a proximal portion of the head part 51, whereby suction can be applied to the lumen portion 511 of the head part 51. By this suction, there is generated a suction force F₂ with which the biological tissue 600 is drawn toward and brought into secure contact with the head part 51 at each suction port 512, whereby the biological tissue 600 is fixed assuredly (see FIGS. 4 to 7).

Note that while the shape of the suction port 512 in plan view is a rectangular shape in this embodiment, this is not restrictive, and the shape may be a circular shape or an elliptic shape, for example.

In addition, a distal end surface 513 of the head part 51 is rounded. This makes it possible to securely prevent the inside of the vaginal cavity 200 from being damaged by the distal end surface 513 of the head part 51 during the time when the head part 51 is inserted into the vaginal cavity 200.

The neck part 52 is a part for supporting the head part 51 from the proximal side of the latter. The neck part 52 is composed of a straight bar-shaped body. Note that the outside diameter of the neck part 52 is preferably smaller than the thickness of the head part 51.

As shown in FIGS. 2 to 7, the seal member 6 is a member for airtight sealing of the urethral lumen 100 in a state where the urethral-insertion member 4 is inserted in the urethral lumen 100. The seal member 6 has a main body portion 61 composed of a small piece, and a bolt 62 for positioning the main body portion 61 relative to the urethral-insertion member 4. Note that various metallic materials such as aluminum, aluminum alloys, stainless steels, etc. or various resin materials can be used as material or materials constituting the main body portion 61 and the bolt 62.

The main body portion 61 is formed with a through-hole 611 piercing therethrough in the thickness direction. The main body portion 41 of the urethral-insertion member 4 is passed through the through-hole 611. This permits the main body portion 61 of the seal member 6 to be moved relative to the main body portion 41 of the urethral-insertion member 4 in the longitudinal direction of the latter. By this movement, the main body portion 61 can cover a urethral orifice 101 by approaching and making secure contact with the urethral orifice 101 (see FIGS. 1 and 2) in a state where the urethral-insertion member 4 is inserted in the urethral lumen 100. It follows that the urethral lumen 100 is securely sealed airtight, and, accordingly, the biological tissue 600 can be assuredly sucked at each of the suction ports 441 of the urethral-insertion member 4.

Note that the main body portion 61 of the seal member 6 is preferably provided, between itself and the main body portion 41 of the urethral-insertion member 4, with a packing formed of an elastic material.

The bolt 62 is in screw engagement with a female screw 612 provided at an outer circumferential portion of the main body portion 61. When the bolt 62 is tightened, the bolt 62 presses and engages the main body portion 41 of the urethral-insertion member 4. This makes it possible to securely prevent the main body portion 61 from unwillingly moving relative to the main body portion 41 of the urethral-insertion member 4, and, therefore, to maintain a state where the urethral lumen 100 is sealed airtight by the main body portion 61. Note that when the bolt 62 is untightened, the main body portion 61 becomes movable again.

A fixing member 7 is a member for fixing a second slide member 8b mounted to the vaginal-insertion member 5, by stopping sliding of the second slide member 8b along a guide groove 92. This fixing member 7 has a main body portion 71 composed of a small piece, and a bolt 72 for positioning the main body portion 71 relative to the vaginal-insertion member 5. Note that various metallic materials such as aluminum, aluminum alloys, stainless steels, etc. or various resin materials can be used as material or materials constituting the main body portion 71 and the bolt 72.

The bolt 72 is in screw engagement with a female screw 712 provided at an outer circumferential portion of the main body portion 71. When the bolt 72 is tightened, the bolt 72 presses and engages the neck part 52. By this, the main body portion 71 is fixed to the neck part 52. When the fixing member 7 is fixed in a state where the fixing member 7 is put in contact with clamping members 9a and 9b from the proximal side, the clamping members 9a and 9b are fixed by being clamped between the proximal side of the second slide member 8b and the distal side of the fixing member 7. As a results of this, the fixing member 7 functions to stop the sliding of the second slide member 8b along the guide groove 92, thereby fixing a vertical spacing between the urethral-insertion member 4 and the vaginal-insertion member 5.

As shown in FIG. 3, the interlock means 30 is means for interlocking the urethral-insertion member 4 and the vaginal-insertion member 5 in parallel. Furthermore, as shown in FIGS. 4 to 7, the interlock means 30 interlocks the rotational support member 2, as well.

As shown in FIG. 12, the interlock means 30 includes: a first slide member 8a mounted to the urethral-insertion member 4; the second slide member 8b mounted to the vaginal-insertion member 5; and one pair of the clamping members 9a and 9b for collectively clamping the first slide member 8a and the second slide member 8b therebetween. Note that various metallic materials such as aluminum, aluminum alloys, stainless steels, etc. or various resin materials can be used as material or materials constituting the first slide member 8a, the second slide member 8b, and the clamping members 9a and 9b.

The first slide member 8a includes: a block-shaped main body portion 81; a bolt 82 for positioning the main body portion 81 relative to the urethral-insertion member 4; and projections 83 formed to project in opposite directions from both side portions of the main body portion 81.

Note that the second slide member 8b is the same in configuration as the first slide member 8a, except that the projections 83 are omitted. In view of this, the configuration of the first slide member 8a will be described on a representative basis.

The main body portion 81 is formed therein with a through-hole 811. With the main body portion 41 of the urethral-insertion member 4 passed through this through-hole 811, the first slide member 8a is mounted to the urethral-insertion member 4. In addition, the main body portion 81 can be moved relative to the main body portion 41 of the urethral-insertion member 4 in the longitudinal direction of the latter. Besides, by this movement, the position of the main body portion 81 relative to the main body portion 41 of the urethral-insertion member 4 can be controlled.

The bolt 82 is in screw engagement with a female screw 812 provided at an outer circumferential portion of the main body portion 81. When the bolt 82 is tightened, the bolt 82 presses and engages the main body portion 41 of the urethral-insertion member 4. By this, the main body portion 81 is positioned relative to the main body portion 41 of the urethral-insertion member 4. Note that when the bolt 82 is untightened, the main body portion 81 becomes movable again.

Each of the projections 83 is a portion which is in the shape of a prism with a tetragonal base and which is formed integrally with the main body portion 81.

As shown in FIG. 12, the clamping members 9a and 9b are each elongated plate-like in shape and are disposed opposite to each other. The clamping member 9a and the clamping member 9b are substantially the same in configuration; in view of this, the configuration of the clamping member 9a will mainly be described on a representative basis.

The clamping member 9a is formed with a through-hole 91 piercing therethrough in the thickness direction. The through-hole 91 is tetragonal in shape in side view. When the clamping member 9a cooperates with the clamping member 9b to clamp the main body portion 81 of the first slide member 8a therebetween, one 83 of the two projections 83 of the first slide member 8a is inserted in the through-hole 91. Note that the other projection 83 of the first slide member 8a is inserted in the through-hole 91 in the clamping member 9b. By such insertion, the first slide member 8a is fixed to the clamping members 9a and 9b.

In addition, the clamping member 9a is formed with the guide groove 92 in its surface on the side of the clamping member 9b (in its surface 95 on the inner side). The guide groove 92 is formed along the vertical direction. In a state where the main body portion 81 of the second slide member 8b is clamped between the clamping member 9a and the clamping member 9b, both side portions of the main body portion 81 of the second slide member 8b are inserted in the respective guide grooves 92 of the clamping members 9a and 9b, and the second slide member 8b can be moved along the guide grooves 92. By this movement, the urethral-insertion member 4 and the vaginal-insertion member 5 can be brought closer to and away from each other (see FIGS. 3 and 4).

Note that both side portions of the main body portion 81 of the second slide member 8b are inserted in the respective guide grooves 92 of the clamping members 9a and 9b, whereby the second slide member 8b is positioned relative to the clamping members 9a and 9b in a direction orthogonal to the guide grooves 92.

The clamping member 9a is formed with a plurality of cylindrical pins 93 projecting from its inner-side surface 95, whereas the clamping member 9b is formed in an inner-side of the surface 95 with guide holes 94 into which the respective pins 93 are inserted. By the fitting of the pins 93 and the guide holes 94 to each other, a clamping state between the clamping member 9a and the clamping member 9b is maintained.

As shown in FIG. 12, outer-side surfaces 96 of the clamping members 9a and 9b are formed with recesses 98 for corresponding engagement with a pair of claws 26 projecting from lower portions of the rotational support member 2. By this engagement, the rotational support member 2 is interlocked to the clamping members 9a and 9b.

Now, a method of using the puncture apparatus 1 will be described below referring to FIGS. 1 to 9 and 13.

Meanwhile, the biological tissue 600 has a part which is suitable for puncturing by the puncture member 3. As an example of such a part, there may ordinarily be mentioned the vicinity 601 of the mid-urethra located between the urethral lumen 100 and the vaginal cavity 200. The position of this mid-urethra varies from person to person; for instance, in some women, the bladder neck is drooping to get comparatively near the urethral orifice 101, so that the mid-urethra itself is also shifted toward the urethral orifice 101, resulting in a shortened mid-urethra. In the case of applying the surgical treatment to such a woman, specifically, in the case of puncturing a vicinity of the mid-urethra with a puncture needle, there may be a risk, depending on the operator's skill, that the puncture needle would be deviated from the vicinity of the mid-urethra, as has been experienced in the past.

In view of this, the puncture apparatus 1 can be suitably used especially for such a woman.
[1] First, the urethral-insertion member 4 is prepared. The urethral-insertion member 4 has the seal member 6 and the first slide member 8a preliminarily mounted thereto. In addition, the seal member 6 is located distally of the first slide member 8a. Besides, the port 45 of the urethral-insertion member 4 is in connection with a wall suction in an operating room.

As shown in FIG. 1, the urethral-insertion member 4 is inserted into the urethral lumen 100. In this instance, such an adjustment is conducted that a marker 46 affixed to the main body portion 41 of the urethral-insertion member 4 is located at the urethral orifice 101 or in front of the urethral orifice 101. This ensures that the balloon 42 is disposed inside the bladder 700. In this instance, besides, the seal member 6 is located apart from the urethral orifice 101.

Note that when performing an operation of inserting the urethral-insertion member 4, the balloon 42 of the urethral-insertion member 4 is set in a contracted state. This permits the inserting operation to be carried out easily.

Then, the balloon 42 is expanded. By this, the balloon 42 is engaged with a bladder neck, so that the urethral-insertion member 4 can be securely prevented from being pulled out of the urethral lumen 100.

[2] Next, the vaginal-insertion member 5 is prepared. The vaginal-insertion member 5 has the second slide member 8b and the fixing member 7 preliminarily mounted thereto. In addition, the second slide member 8b is located distally of the fixing member 7. Besides, the port 53 of the vaginal-insertion member 5 is also in connection with the wall suction in the operating room.

As shown in FIG. 2, the head part 51 of the vaginal-insertion member 5 is substantially wholly inserted into the vaginal cavity 200.

In this inserted state, each of the suction ports 512 of the head part 51 is facing the biological tissue 600. Note that each suction port 512 may be located apart from the biological tissue 600 as in the state shown in FIG. 2, or may instead be in contact with the biological tissue 600.

[3] Subsequently, as shown in FIG. 3, the seal member 6 mounted to the urethral-insertion member 4 is moved distally, to seal the urethral orifice 101 with the seal member 6. Thereafter, the bolt 62 of the seal member 6 is tightened, to perform positioning relative to the urethral-insertion member 4. This ensures that a state where the urethral orifice 101 is sealed is maintained.

Next, the clamping members 9a and 9b are prepared.

Using the clamping members 9a and 9b, the first slide member 8a mounted to the urethral-insertion member 4 and the second slide member 8b mounted to the vaginal-insertion member 5 are clamped therebetween. As a result, the urethral-insertion member 4 and the vaginal-insertion member 5 are interlocked in parallel. Note that the clamping state between the clamping member 9a and the clamping member 9b is maintained by the fitting between the pins 93 of the clamping member 9a and the guide holes 94 in the clamping member 9b, as aforementioned.

[4] Next, the puncture member 3 and the rotational support member 2 are prepared. The puncture member 3 is rotatably supported on the rotational support member 2.

As shown in FIG. 4, the rotational support member 2 is interlocked to the clamping members 9a and 9b in the clamping state. Note that the rotational support member 2 is preferably located slightly distally of the clamping members 9a and 9b. In this instance, besides, the needle tip 315 of the puncture member 3 is deviated from a position directly above the biological tissue 600, and is located directly above the bladder 700.

Subsequently, the biological tissue 600 is sucked (fixed) at the suction ports 441 of the urethral-insertion member 4, and the biological tissue 600 is sucked (fixed) at the suction ports 512 of the vaginal-insertion member 5. Then, keeping this suction state (fixation state), the vaginal-insertion member 5 is lowered downward so that the urethral-insertion member 4 and the vaginal-insertion member 5 are spaced away from each other. It follows that the biological tissue 600 is expanded in the thickness direction thereof (in the vertical direction).

In addition, the operation of lowering the vaginal-insertion member 5 is performed until the neck part 52 of the vaginal-insertion member 5 is moved past a marker 97 affixed to the outer-side surface 96 of the clamping member 9a. As a result, the separated distance between the urethral-insertion member 4 and the vaginal-insertion member 5 is so set that the biological tissue 600 is expanded to such an extent that it can be assuredly punctured by the puncture member.

Besides, in the state where the urethral-insertion member 4 and the vaginal-insertion member 5 are spaced with this separated distance therebetween, the fixing member 7 is put into contact with (pressed against) the clamping members 9a and 9b, and the bolt 72 is tightened. By this, the urethral-insertion member 4 and the vaginal-insertion member 5 are fixed in the spaced state.

[5] Next, as shown in FIG. 5, keeping the positions of the puncture member 3 and the rotational support member 2 the same as during the above operation [4], the clamping members 9a and 9b are grasped and the puncture apparatus 1 as a whole is pressed distally. It follows that the urethral-insertion member 4 is pressed in the insertion direction thereof, and the vaginal-insertion member 5 is pressed in the insertion direction thereof. In other words, the urethral-insertion member 4 and the vaginal-insertion member 5 are pressed in the same direction, in parallel. By such a pressing, the urethral lumen 100 and the vaginal cavity 200 are moved distally, and the biological tissue 600 is pushed in in the same direction as the pressing direction. As a result, a puncture orbital path for the puncture member 3 is located above the vicinity 601 of the mid-urethra, which is part of the biological tissue 600.

[6] Subsequently, as shown in FIG. 6, in the state where the biological tissue 600 has been pushed in, a grasping unit 34 provided at a proximal portion of the puncture member 3 is grasped, and the puncture member 3 is rotationally moved. The rotating direction is a direction for permitting puncturing of the vicinity 601 of the mid-urethra with the needle tip 315 of the puncture member 3, that is, clockwise in FIG. 13. In addition, the limit for the rotary movement of the puncture member 3 is until the interlock portion 32 of the puncture member 3 comes into contact with the patient's body surface. Such a rotary movement causes the needle tip 315 of the puncture member 3 to enter the patient's body via the body surface 800 in the patient's right-hand inguinal region or in the vicinity thereof, to pass an obturator foramen 400a of a pelvis 300, the vicinity 601 of the mid-urethra, and another obturator foramen 400b of the pelvis 300 in this order, and to exit the patient's body via the body surface 800 in the left-hand inguinal region or in the vicinity thereof.

[7] Next, as shown in FIG. 7, the puncture member 3 is rotationally moved in the direction opposite to the aforementioned direction. As a result, the through-hole 500 along the circular arc of the puncture needle 31 of the puncture member 3 is formed. In addition, the through-hole 500 naturally is penetrating the vicinity 601 of the mid-urethra.

[8] Subsequently, as shown in FIG. 8, the puncture apparatus 1 is removed from the patient. It follows that the pushing-in force on the biological tissue 600 is canceled, so that the biological tissue 600 is restored by its own restoring force into its original state, namely, the state without the puncture apparatus 1 being mounted onto the patient. In this instance, besides, the through-hole 500 is in a curved form in side view.

[9] Next, as shown in FIG. 9, the band 80 is passed through the through-hole 500 by use of a guide wire (not shown), for example. The band 80 is in the state of hooking the urethra wall, with both end portions thereof protruding from the through-hole 500 to the outside of the patient's body. Then, both end portions of the band 80 which are protruding to the outside of the patient's body are pulled with predetermined forces. As a result, due to a tension on the band 80, the urethral wall is pulled in a direction for spacing away from the vaginal wall, and the urethral lumen 100 is supported by the band 80.

Thereafter, unnecessary portions of the band 80 are cut away, and predetermined wound closure and the like are carried out, to complete the procedure.

The use of the puncture apparatus 1 as aforementioned ensures that when a surgical treatment of forming a through-hole 500 for placing a band 80 indwelling is applied to a biological tissue 600 located between a urethral lumen 100 and a vaginal cavity 200, particularly to the vicinity 601 of the mid-urethra, the treatment can be carried out in an assured manner.

### <Second Embodiment>

FIG. 14 shows vertically sectional side views for sequentially illustrating a method of using a puncture apparatus of the present invention (a second embodiment).

Hereafter, a second embodiment of the puncture apparatus of the present invention will be described referring to this drawing. The following description will center on differences from the aforementioned embodiment, and descriptions of the same items as above will be omitted.

This embodiment is the same as the first embodiment, except for a difference in the configuration of the biological tissue pushing-in means.

In a puncture apparatus 1 in this embodiment as shown in FIG. 14, unlike in the first embodiment above, biological tissue pushing-in means 20 has a urethral-insertion member 4, with a vaginal-insertion member 5 omitted therefrom. The urethral-insertion member 4 is inserted into a urethral lumen 100, and a part on the deeper side of a bladder 700 is pressed by the urethral-insertion member 4 further in the insertion direction, whereby a biological tissue 600, particularly a vicinity 601 of a mid-urethra where a puncture hole 500 is to be formed, can be pushed in. In this pushed-in state, a puncture member 3 is operated, whereby the puncture hole 500 can be formed in the vicinity 601 of the mid-urethra in an assured manner.

### <Third Embodiment>

FIG. 15 shows vertically sectional side views for sequentially illustrating a method of using a puncture apparatus of the present invention (a third embodiment).

Hereafter, a third embodiment of the puncture apparatus of the present invention will be described referring to this drawing. The following description will center on differences from the aforementioned embodiment, and descriptions of the same items as above will be omitted.

This embodiment is the same as the first embodiment above, except for a difference in the configuration of the biological tissue pushing-in means.

In a puncture apparatus 1 in this embodiment as shown in FIG. 15, unlike in the first embodiment above, biological tissue pushing-in means 20 has a vaginal-insertion member (elongated member) 5A, with a urethral-insertion member 4 omitted therefrom. The vaginal-insertion member 5A is elongated in shape, and has a bent portion 54 formed at a distal portion thereof. In addition, on the distal side of and in the vicinity of the bent portion 54, there is formed a flange 55 that has an enlarged outside diameter.

The vaginal-insertion member 5A configured in this fashion is inserted into a vaginal cavity 200, and a cervical zone 900 of a uterus is pressed further in the insertion direction by the vaginal-insertion member 5A, whereby a biological tissue 600, particularly a vicinity 601 of a mid-urethra where a puncture hole 500 is to be formed, can be pushed in. In this pushed-in state, a puncture member 3 is operated, whereby the puncture hole 500 can be formed in the vicinity 601 of the mid-urethra in an assured manner.

While the puncture apparatus of the present invention has been described hereinabove with reference to the embodiments shown in the drawings, the invention is not limited to the embodiments. Each of the components of the puncture apparatus can be replaced with one having an arbitrary configuration that is able to exhibit a similar function. Besides, arbitrarily configured bodies may be added.

In addition, the puncture apparatus of the present invention may be a combination of arbitrary two or more configurations (features) of the embodiments above.

Besides, while description in the first embodiment above has been made with the urethral-insertion member side as "primary" and with the vaginal-insertion member side as "secondary (auxiliary)," this is not restrictive; the vaginal-insertion member side may be taken as "primary" and the urethral-insertion member side may be taken as "secondary (auxiliary)."

While the puncture needle has been a curved one in the first embodiment above, this is not restrictive; for example, a rectilinear puncture needle may also be adopted.

The urethral-insertion member may be provided with a urine drain lumen through which urine in a bladder is to be discharged.

In the first embodiment above, there have been formed a plurality of the suction ports functioning as a fixing part for fixing the biological tissue from the urethral lumen side, and there have been formed a plurality of the suction ports functioning as a fixing part (auxiliary fixing part) for fixing the biological tissue from the vaginal cavity side. However, this configuration is not restrictive. For example, only one suction port of the first kind and only one suction port of the second kind may be formed.

In addition, while the fixing parts have been configured to achieve fixation by suction in the first embodiment above, this is not restrictive. For instance, the fixing parts may be configured to achieve fixation by clamping.

Besides, while description herein has been made assuming that the puncture apparatus of the present invention is applied to a female patient, the puncture apparatus of the present invention can also be applied to a male by a method in which an elongated member is inserted into a lumen artificially formed by incision of the perineal region or into the rectum, instead of into the vagina.

### Industrial Applicability

The puncture apparatus of the present invention includes: puncture means which has a puncture needle for puncturing a biological tissue located in a vicinity of a mid-urethra; and biological tissue pushing-in means which has an elongated member having an elongated shape to be inserted into one of a urethral lumen and a vaginal cavity, the elongated member being inserted into the one of the urethral lumen and the vaginal cavity and being pressed in the insertion direction thereof, whereby the biological tissue is pushed in in the same direction as the pressing direction for the elongated member. The puncture apparatus is used by puncturing the biological tissue with the puncture needle in a state where the biological tissue has been pushed in by the biological tissue pushing-in means. Therefore, the puncture apparatus ensures that when a surgical treatment is applied to a biological tissue located between a vaginal cavity and a urethral lumen, the treatment can be carried out in an assured manner.

### Description of Reference Symbols

1: Puncture apparatus
2: Rotational support member
25: Pass-through hole
26: Claw
3: Puncture member
31: Puncture needle
315: Needle tip
32: Interlock portion
33: Shaft portion
331, 332: Flange
34: Grasping unit
4: Urethral-insertion member (elongated member)
41: Main body portion
42: Balloon
43: Inner tube
431: Lumen portion
44: Outer tube
441: Suction port
442: Lumen portion
443: Distal end surface
45: Port
46: Marker
5: Vaginal-insertion member (auxiliary elongated member)
5A: Vaginal-insertion member (elongated member)
51: Head part
511: Lumen portion
512: Suction port
513: Distal end surface
52: Neck part
53: Port
54: Bent portion
55: Flange
6: Seal member
61: Main body portion
611: Through-hole
612: Female screw
62: Bolt
7: Fixing member
71: Main body portion
712: Female screw
72: Bolt
8a: First slide member
8b: Second slide member
81: Main body portion
811: Through-hole
812: Female screw
82: Bolt
83: Projection
9a, 9b: Clamping member
91: Through-hole
92: Guide groove
93: Pin
94: Guide hole
95: Inner-side surface
96: Outer-side surface
97: Marker
98: Recess
10: Puncture means
20: Biological tissue pushing-in means
30: Interlock means
80: Band
100: Urethral lumen (one of urethral lumen and vaginal cavity)
101: Urethral orifice
200: Vaginal cavity (the other of urethral lumen and vaginal cavity)
300: Pelvis
400a, 400b: Obturator foramen
500: Through-hole (puncture hole)
600: Biological tissue
601: Vicinity of mid-urethra
700: Bladder
800: Body surface
900: Cervical zone of uterus
F₁, F₂: Suction force

## Claims

1. A puncture apparatus comprising:
puncture means which has a puncture needle for puncturing a biological tissue located in a vicinity of a mid-urethra; and
biological tissue pushing-in means which has an elongated member having an elongated shape to be inserted into one of a urethral lumen and a vaginal cavity, the elongated member being inserted into the one of the urethral lumen and the vaginal cavity and being pressed in an insertion direction thereof, whereby the biological tissue is pushed in in the same direction as a pressing direction for the elongated member,
**characterized in that** the puncture apparatus is used by puncturing the biological tissue with the puncture needle in a state where the biological tissue has been pushed in by the biological tissue pushing-in means.

2. The puncture apparatus according to claim 1, wherein the biological tissue pushing-in means has a fixing part which is provided on the elongated member and which fixes the biological tissue to the elongated member from the side of the one of the urethral lumen and the vaginal cavity.

3. The puncture apparatus according to claim 2, wherein the fixing part is a part for sucking the biological tissue to bring the biological tissue into secure contact with the elongated member.

4. The puncture apparatus according to claim 3, wherein the elongated member is a hollow body and is connected to suction means for applying suction to the inside of the hollow body, and
the elongated member is formed with at least one suction port opening in an outer circumferential surface thereof, the suction port functioning as the fixing part and communicating with the inside of the hollow body, with the biological tissue being drawn toward and brought into secure contact with the elongated member by a suction force generated at the suction port when the suction means is operated.

5. The puncture apparatus according to claim 4, wherein the one of the urethral lumen and the vaginal cavity is the urethral lumen, and
the biological tissue pushing-in means has a seal member for airtight sealing of the urethral lumen in a state where the elongated member is inserted in the urethral lumen.

6. The puncture apparatus according to any one of claims 1 to 5, wherein the biological tissue pushing-in means has an auxiliary elongated member having an elongated shape to be inserted into the other of the urethral lumen and the vaginal cavity, the auxiliary elongated member being inserted into the other of the urethral lumen and the vaginal cavity and being pressed in an insertion direction thereof, whereby the biological tissue is pushed in together with the elongated member.

7. The puncture apparatus according to claim 6, wherein the biological tissue pushing-in means has an auxiliary fixing part which is provided on the auxiliary elongated member and which fixes the biological tissue to the auxiliary elongated member from the side of the other of the urethral lumen and the vaginal cavity.

8. The puncture apparatus according to claim 7, wherein the auxiliary fixing part is a part for sucking the biological tissue to bring the biological tissue into secure contact with the auxiliary elongated member.

9. The puncture apparatus according to claim 8, wherein the auxiliary elongated member is a hollow body and is connected to auxiliary suction means for applying suction to the inside of the hollow body, and
the auxiliary elongated member is formed with at least one auxiliary suction port opening in an outer circumferential surface thereof, the auxiliary suction port functioning as the auxiliary fixing part and communicating with the inside of the hollow body constituting the auxiliary elongated member, with the biological tissue being drawn toward and brought into secure contact with the auxiliary elongated member by a suction force generated at the auxiliary suction port when the auxiliary suction means is operated.

10. The puncture apparatus according to any one of claims 6 to 9, further comprising interlock means for interlocking the elongated member and the auxiliary elongated member so as to permit the elongated member and the auxiliary elongated member to come closer to and away from each other.

11. The puncture apparatus according to claim 10, wherein the biological tissue pushing-in means includes a fixing part which is provided on the elongated member and which fixes the biological tissue to the elongated member from the side of the one of the urethral lumen and the vaginal cavity, and an auxiliary fixing part which is provided on the auxiliary elongated member and which fixes the biological tissue to the auxiliary elongated member from the side of the other of the urethral lumen and the vaginal cavity, and
the interlock means is so configured as to be capable of spacing the elongated member and the auxiliary elongated member away from each other and maintaining the spaced-apart state, in a state where the biological tissue is fixed to the elongated member by the fixing part and where the biological tissue is fixed to the auxiliary elongated member by the auxiliary fixing part.

12. The puncture apparatus according to claim 11, further comprising a maker which, when the elongated member and the auxiliary elongated member are spaced away from each other, indicates the separated distance between the elongated member and the auxiliary elongated member.

13. The puncture apparatus according to any one of claims 1 to 12, wherein the puncture needle is curved in a circular arc shape, and
the puncture means has a rotational support member for supporting the puncture needle so as to permit rotary movement of the puncture needle about a center of the circular arc.
